# EUROPÄISCHE PATENTSCHRIFT

(11) **EP 3 010 827 B1**
(45) Veröffentlichungstag und Bekanntmachung des Hinweises auf die Patenterteilung: **26.04.2017**
(21) Anmeldenummer: 14722674.0
(22) Anmeldetag: 08.05.2014
(51) Int. Cl.: B65D 83/00

(54) **SPENDER FÜR FLÜSSIGKEITEN**
DISPENSER FOR LIQUIDS
DISTRIBUTEUR POUR LIQUIDES

(30) Priorität: 18.06.2013 DE 102013211423
(43) Veröffentlichungstag der Anmeldung: 27.04.2016
(73) Patentinhaber: Aptar Radolfzell GmbH, 78315 Radolfzell (DE)
(72) Erfinder: WOCHELE, Matthias, 71292 Friolzheim (DE)
(74) Vertreter: Patentanwaltskanzlei Cartagena
(86) Internationale Anmeldenummer: PCT/EP2014/059498
(87) Internationale Veröffentlichungsnummer: WO 2014/202278

(56) Entgegenhaltungen:
- EP-A2- 1 985 543
- WO-A1-2012/169489
- FR-A1- 2 389 419
- US-A- 5 344 045
- US-A- 5 373 967
- US-A1- 2005 274 687
- US-A1- 2009 289 073
- US-A1- 2010 075 001
- US-A1- 2010 147 726

## Beschreibung

Die Erfindung betrifft einen Spender zum Austrag eines fließfähigen pharmazeutischen oder kosmetischen Mediums mit einem Austragkopf mit Austragöffnung und mit einem Behälter zur Bevorratung des fließfähigen Mediums vor dem Austrag. Der Behälter verfügt dabei über einen Außenbehälter und einen in dessen Innenraum angeordneten Innenbehälter. Der Außenbehälter ist elastisch verformbar, worunter zu verstehen ist, dass er in Art einer Quetschflasche durch einen Benutzer zusammengedrückt werden kann, um sein Innenvolumen zu reduzieren und hierdurch Flüssigkeit auszutragen, und dass der Außenbehälter nach Wegfall der Kraftbeaufschlagung in seine Ausgangsform zurückkehrt. Der im Außenbehälter angeordnete Innenbehälter ist der das Medium unmittelbar aufnehmende Teilbehälter. Zwischen diesem Innenbehälter und dem Außenbehälter ist ein Freiraum vorgesehen, welcher mit zunehmender Entleerung des Innenbehälters kleiner wird, da das Außenvolumen des Innenbehälters durch Entnahme von Medium sinkt, während das Innenvolumen des Außenbehälters konstant bleibt.

Um einen zu Störungen beim Betrieb des Spenders führenden Unterdruck in diesem Freiraum zu vermeiden, ist bei gattungsgemäßen Spendern ein Belüftungskanal vorgesehen, durch den der Innenraum des Außenbehälters mit einer umgebenden Atmosphäre zum Zwecke des Druckausgleichs mittels Ausgleichsluft verbunden ist.

Der genannte Belüftungskanal gestattet es, nach erfolgtem Austrag Luft aus der Umgebung in den genannten Freiraum einzusaugen, so dass im Behälter anschließend wieder in etwa der Umgebungsdruck herrscht. Der Belüftungskanal ist jedoch grundsätzlich auch problematisch, da er bei einem bestimmungsgemäßen Zusammendrücken des Außenbehälters zum Zwecke des Druckaufbaus auf den Innenbehälter und somit zum Zwecke des Austrags dem Druckaufbau entgegenwirkt. Je langsamer die Betätigung durch Zusammendrücken des Außenbehälters erfolgt, desto mehr Luft kann durch den Belüftungskanal nach außen entweichen und somit die Menge des auszutragenden Mediums verringern, da der im Freiraum entstehende Druck nicht in der gewünschten Höhe aufgebaut wird.

Die gattungsgemäße EP 1 985 543 A2 wirkt diesem Problem dadurch entgegen, dass der Belüftungskanal dafür vorgesehen ist, vom Benutzer mittels eines seiner Finger während der Betätigung verschlossen zu werden. Dies führt allerdings zu einer erschwerten Handhabung.

Aufgabe der Erfindung ist es, eine technische Lösung zur Verfügung zu stellen, mittels derer auf konstruktiv einfache Art und Weise ein Belüftungskanal geschaffen werden kann, dessen Strömungswiderstand so bemessen ist, dass bei einem Zusammendrücken des Außenbehälters ein zum Austragen des Mediums erforderlicher Druck im Innenbehälter aufgebaut werden kann.

Diese Aufgabe wird erfindungsgemäß durch einen Spender gemäß Anspruch 1 gelöst.

US 5373967 und EP 1985543 A2 offenbaren einen Spender gemäß dem Oberbegriff des Anspruchs 1.

Gemäß einem der Erfindung ist dieser Belüftungskanal, worunter auch eine einfache Belüftungsöffnung zu verstehen ist, zumindest abschnittsweise durch das genannte Etikett, welches insbesondere vorzugsweise als selbstklebendes Etikett ausgebildet ist, begrenzt. Wie bereits erwähnt, bewirkt das Etikett einen Strömungswiderstand im Belüftungskanal, der ausreichend hoch ist, um den bestimmungsgemäßen Aufbau des zum Austrag erforderlichen Drucks im Innenbehälter zu gestatten.
Das zumindest abschnittsweise Begrenzen des Belüftungskanals durch ein insbesondere selbstklebendes Etikett ist dahingehend zu verstehen, dass das Etikett so angebracht ist, dass es durch seine Anbringung den Strömungswiderstand erhöht, wobei gemäß diesem ersten Aspekt der Erfindung durch das Etikett ein freier Pfad für die Luft makroskopisch unverschlossen bleibt. Verschiedene Anbringungsarten hierzu werden im Weiteren noch erläutert. Allgemein wird es als vorteilhaft angesehen, wenn das Etikett auf der Außenseite einer von außen zugänglichen Oberfläche des Spenders angebracht ist, insbesondere auf dem genannten Außenbehälter.
Die Verwendung eines Etiketts ist deswegen besonders von Vorteil, da sie es gestattet, den Strömungswiderstand auf einfache Weise zu beeinflussen und durch die Auswahl des Etikettes bzw. den Anbringungsort flexibel zu beeinflussen und somit abhängig vom konkreten Anwendungszweck die Charakteristik des Belüftungskanals zu beeinflussen. Des Weiteren ist es von Vorteil, wenn das Etikett nicht nur dem Zweck der Beeinflussung des Strömungswiderstandes dient, sondern gleichzeitig auch ein Beschriftungsetikett darstellt, auf dem Benutzerinformationen stehen, wie insbesondere der Name des Herstellers des Spenders bzw. der Kosmetik oder des Pharmazeutikums, Verwendungshinweise, Zusammensetzungen des Inhalts des Spenders und dergleichen.

Das im genannten Kontext verwendete Etikett ist ein folienartiger dünner Flächenabschnitt mit einer Dicke von weniger als 300 µm, vorzugsweise weniger als 200 µm. Dieses Etikett ist auf einer Fläche aufgebracht und wirkt dort den Strömungswiderstand des Belüftungskanals beeinflussend. Es handelt sich vorzugsweise um ein selbstklebendes Etikett. Zwar sind grundsätzlich auch Gestaltungen denkbar, bei denen der Kleber zur Anbringung des Etiketts separat vor Anbringung des Etiketts an der genannten Fläche aufgebracht wird, eine selbstklebende Gestaltung ist jedoch von Vorteil. In Einzelfällen können auch andere Anbringungsmethoden für das Etikett vorteilhaft sein, so insbesondere umlaufende und aufgeschrumpfte Etiketten oder aufgeschweißte oder anderweitig thermisch befestigte Etiketten.

Der Außenbehälter kann beispielsweise die Form einer Flasche oder einer Tube aufweisen. Er ist insbesondere lösbar mit einem Austragkopf verbunden, an dem die Austragöffnung vorgesehen ist. Diese ist vorzugsweise vor Betätigung des Spenders mittels eines druckabhängig öffnenden Ventils verschlossen.

Eine Gestaltung sieht vor, dass ein Kanalabschnitt des Belüftungskanals gemeinsam gebildet wird durch eine nutartig langgestreckte Vertiefung in einem Kanalbauteil, wobei es sich wiederum vorzugsweise um den Außenbehälter handelt, und dass das Etikett auf dem Kanalbauteil aufgebracht ist, so dass der Kanalabschnitt durch die Vertiefung und das Klebeetikett umfänglich geschlossen ist.

Es ist somit vorgesehen, dass eine Nut, die insbesondere in einer Außenseite des Außenbehälters vorgesehen sein kann, durch das aufgebrachte Etikett zu einem umfänglich geschlossenen Kanal ergänzt wird. Der Strömungswiderstand des Belüftungskanals kann so sehr einfach erhöht werden. Wenn ein besonders großer Strömungswiderstand gewünscht ist, wird der Querschnitt der Vertiefung sehr klein gewählt. Alternativ oder zusätzlich kann auch eine besonders lange Vertiefung Verwendung finden, welche beispielsweise auch durch eine spiralförmige oder helixförmige Formgebung des Belüftungskanals auf einfache Weise erzielt werden kann. Der umgebungsseitige Einlass des Belüftungskanals wird durch das Ende des Kanalabschnittes gebildet, der gemeinsam durch die Vertiefung und das Etikett erzeugt wird.

Wie eingangs bereits erwähnt wurde, geht es bei der Gestaltung des Belüftungskanals bzw. der Belüftungsöffnung insbesondere darum, den diesbezüglichen Strömungswiderstand so zu gestalten, dass sich beim Zusammendrücken des Außenbehälters ein zum Austragen des Mediums ausreichender Druck auf den Innenbehälter aufbaut. Dies ist vorzugsweise dadurch realisiert, dass der Belüftungskanal einen Strömungswiderstand aufweist, der ausreichend hoch ist, als dass bei einer Druckdifferenz von 700 mbar zwischen dem Außenbehälter und der Umgebung der Volumenstrom 0,2 ml/s nicht übersteigt. Es wurde festgestellt, dass ein solcher Strömungswiderstand, der bei einem absoluten Innendruck von etwa 1,7 bar gegenüber einem Umgebungsdruck von etwa 1 bar lediglich 0,2 ml/s aus dem Zwischenraum zwischen Innenbehälter und Außenbehälter ausströmen lässt, übliche Betätigungen nicht in störendem Maße negativ beeinflusst. Näherungsweise und unter Nichtbeachtung der Veränderung der Einflussgrößen führt also ein Überdruck im Freiraum zwischen Innenbehälter und Außenbehälter gegenüber der umgebenden Atmosphäre von 0,7 bar bei einer Betätigungszeit von 5 Sekunden zu einem maximalen Luftverlust von 1ml aus dem Freiraum zwischen Innenbehälter und Außenbehälter. Ein zu großer Strömungswiderstand ist jedoch ebenfalls nicht gewünscht, da dies die Dauer für den Druckausgleich zu stark erhöhen würde. Um bei üblichen Intervallen beispielsweise zum Austragen mehrerer Chargen bei einem Tropfenspender den Spender schnell wieder verwenden zu können, insbesondere nach 15 - 30 s, ist es von Vorteil, wenn bei einer Druckdifferenz von 200 mbar der Volumenstrom 0,2 ml/s beträgt.
Insgesamt ist somit eine Gestaltung des Belüftungskanals von Vorteil, die einen Volumenstrom von 0,2 ml/s bei einer Druckdifferenz gestattet, die zwischen 200 mbar und 700 mbar liegt. Es ist allerdings zu beachten, dass der Strömungswiderstand für das Einströmen von Luft in den Freiraum und für das Ausströmen von Luft aus dem Freiraum je nach Geometrie des Belüftungskanals nicht identisch sein muss. Verallgemeinert ist der Strömungswiderstand des Belüftungskanals derart ausgestaltet, dass beim Ausströmen von 0,2 ml/sek Luft ein Überdruck von 700 mbar vorliegen muss und/oder dass für das Einströmen von 0,2 ml/sek Luft ein Unterdruck von 200 mbar vorliegen muss. Der Belüftungskanal ist erfindungsgemäß zumindest abschnittsweise als Kapillarkanal ausgebildet. Dieser weist eine minimale Querschnittsfläche auf, die kleiner als 1 mm² ist. Insbesondere von Vorteil ist es, dass der Quotient aus der Länge des Kapillarkanals und der mittleren Querschnittsfläche des Kapillarkanals in jenem Bereich, in dem die Querschnittsfläche kleiner als 1 mm² ist, größer als 200 mm⁻¹ ist.
Die Verwendung eines Etiketts, insbesondere eines selbstklebenden Etikettes, kann in der zuvor beschriebenen Art derart erfolgen, dass das Etikett den Strömungswiderstand des Belüftungskanals dadurch beeinflusst, dass dessen Länge beeinflusst wird oder dadurch, dass dessen minimaler Querschnitt beeinflusst wird.

Der Spender ist in vorteilhaften Ausgestaltungen als Tropfenspender, insbesondere als Augentropfenspender ausgeführt. Insbesondere handelt es sich um einen Spender, insbesondere Tropfenspender, zur Abgabe von Arzneimitteln, die Formulierungen in flüssiger Darreichungsform von in der Augenheilkunde (Behandlung des erhöhten Augeninndrucks, Glaukombehandlung, Behandlung des trockenen Auges, Behandlung von Allergien und Entzündungen) gängigen Molekülgruppen enthalten. Dabei handelt es sich vor allem um die Molekülgruppen Alpha-2-Agonisten, (z. B. Brimonidin), Prostaglandinanaloga (z.B. Tafluprost, Latanoprost, Bimatoprost, Travoprost), Beta-Blocker (z. B. Timolol) und um Carboanhydrasehemmer (z. B. Dorzolamid), weiterhin um Hyaluronsäureverbindungen, Filmbildner allgemein (z. B. Methylcelluloseverbindungen) und um Cyclosporin sowie um Antihistaminika (z. B. Olopatadin und Levocabastin), Steroide (z. B. Loteprednol und Dexamethason) und um NSAID (z. B. Keterolac).

### Kurzbeschreibung der Zeichnungen

Weitere Aspekte und Vorteile der Erfindung ergeben sich aus den Ansprüchen sowie der nachfolgenden Beschreibung bevorzugter Ausführungsbeispiele der Erfindung, die anhand der Figuren erläutert werden. Dabei zeigen:
- Fig. 1: einen Spender, der in erfindungsgemäßer Art mit Außenbehälter und Innenbehälter versehen ist,
- Fig. 2A bis 2D: den Außenbehälter und Innenbehälter einer ersten Ausführungsform eines erfindungsgemäßen Spenders,
- Fig. 3A: eine Variante zum Behälter der Figuren 2A bis 2D.

### Detaillierte Beschreibung der Ausführungsbeispiele

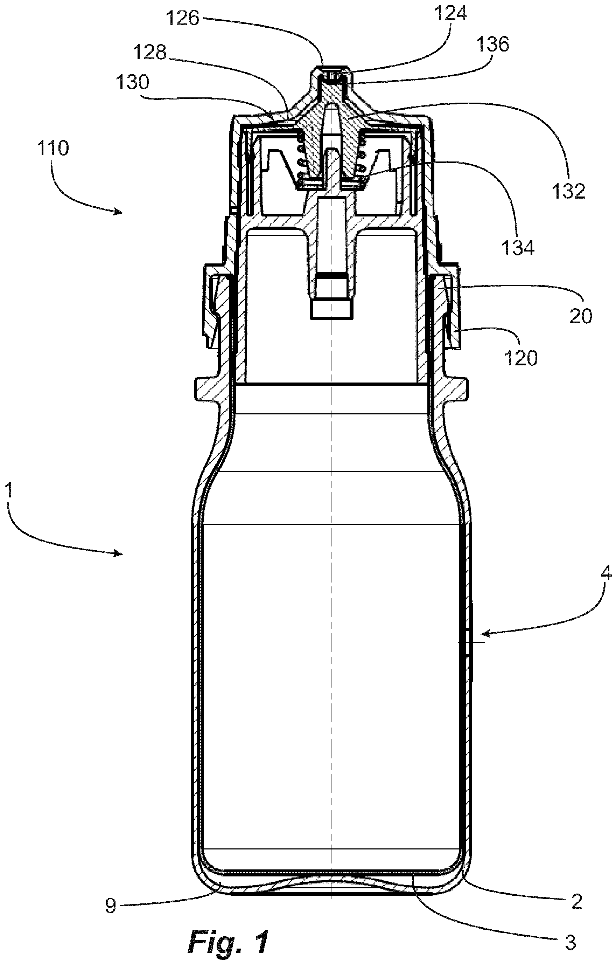

Die Fig. 1 zeigt einen erfindungsgemäßen Spender in einer geschnittenen Darstellung. Dabei ist der hier vorgesehene Belüftungskanal bei dem Spender der Fig. 1 lediglich exemplarisch zu verstehen. Verschiedene Ausgestaltungen des Belüftungskanals lassen sich im Detail den weiteren Darstellungen entnehmen.

Der erfindungsgemäße Spender gemäß Fig. 1 verfügt über einen Austragkopf 110, welcher auf einen Behälter 1 mittels beidseitiger Rastmittel 20, 120 aufgerastet ist. Der Austragkopf 110 verfügt in an sich bekannter Art und Weise über ein Auslassventil 130 mit einem durch eine Ventilfeder 134 kraftbeaufschlagten Ventilkörper 132. Dieser Ventilkörper 132 verschließt eine Austragöffnung 124 mittels einer Ventilfläche 136.

Außenseitig der Austragöffnung 124 ist eine Tropfenbildungsfläche 126 vorgesehen. Zwischen dem Ventilkörper 132 und einer Innenseite des Gehäuses des Austragkopfes 110 ist ein Ventilraum 128 vorgesehen. Wenn in einer Überkopflage, in der der Austragkopf 110 nach unten weist, die Flüssigkeit im Behälter 1 durch manuelles Zusammendrücken des Behälters 1 druckbeaufschlagt wird, so führt dies auch zu einer Druckerhöhung im Ventilraum 128. Gegen die Kraft der Ventilfeder 134 wird in Reaktion hierauf der Ventilkörper 132 verlagert und gibt einen Flüssigkeitspfad zur Austragöffnung 124 frei. Es kommt dann zum Flüssigkeitsaustrag.

Vorliegend ist der Spender als Tropfenspender ausgestaltet und daher mit der bereits genannten Tropfenbildungsfläche 126 versehen. Die durch die Austragöffnung 124 ausströmende Flüssigkeit sammelt sich an dieser Tropfenbildungsfläche, bis sie sich in Form eines Tropfens hiervon löst.

Die Ausgestaltung als Tropfenspender ist exemplarisch zu verstehen. Auch andere Spendertypen, beispielsweise solche, die einen Sprühstrahl erzeugen, können in erfindungsgemäßer Art und Weise ausgestaltet sein.

Der Behälter 1 ist mehrlagig ausgebildet. Er verfügt über einen Außenbehälter 2 aus einem elastischen Kunststoff, der eine Quetschflasche bildet. Dies bedeutet, dass bestimmungsgemäß zum Austrag von Flüssigkeit der Außenbehälter 2 zusammengedrückt wird, beispielsweise mit einer Kraft zwischen 5 N und 20 N. Entfällt diese Kraft, so kehrt der Außenbehälter unmittelbar oder nach einer kurzen Zeitspanne in seine in Fig. 1 dargestellte Ausgangsform zurück. Innerhalb des Außenbehälters 2 ist ein Innenbehälter 3 angeordnet. Hierbei handelt es sich um einen formflexiblen Beutel, der zur Aufnahme der auszutragenden Flüssigkeit vorgesehen ist. Hierbei handelt es sich vorzugsweise um eine pharmazeutische oder kosmetische Flüssigkeit, vorliegend insbesondere um ein in Form von Augentropfen zu applizierendes Medikament. Anders als beim Außenbehälter 2 ist beim beutelartigen Innenbehälter 3 nicht vorgesehen, dass dieser sich nach Ende eines Austragvorgangs volumetrisch wieder vergrößert. Stattdessen ist vorgesehen, dass das Volumen des Innenbehälters sich mit fortschreitender Entleerung des Spenders verringert, ohne dass es hierbei zu nennenswerten Rückstellkräften in den Wandungen des Innenbehälters kommt.

Es ist daher vorgesehen, dass ein Freiraum 9 zwischen dem Außenbehälter 2 und dem Innenbehälter 3 mit fortschreitender Entleerung des Spenders an Volumen gewinnt. Wenn dieser Freiraum gegenüber einer umgebenden Atmosphäre isoliert wäre, so würde sich ein Unterdruck einstellen, der den Austrag von Flüssigkeit unterbinden würde, lange bevor das Medium im Innenbehälter 3 aufgebraucht ist.

Um einen Druckausgleich im Freiraum 9 zu ermöglichen, ist eine Belüftungsöffnung/ein Belüftungskanal 4 vorgesehen. Dieser verbindet den Freiraum 9 mit einer umgebenden Atmosphäre. Da jedoch dieser Druckausgleich es bei bereits teilweise entleertem Innenbehälter 3 verhindern würde, dass sich im Freiraum ein für den Austrag erforderlicher Überdruck bei Betätigung aufbaut, ist der Belüftungskanal 4 so geartet, dass er einen ausreichend hohen Strömungswiderstand aufweist, um dennoch einen problemlosen Austrag zu ermöglichen.

Wie hoch dieser Strömungswiderstand sein sollte, hängt von einer Reihe von Faktoren ab, wie beispielsweise dem Innenvolumen des Außenbehälters und des Innenbehälters. Insbesondere bei Tropfenspendern üblicher Größe, die meist ein Innenvolumen von etwa 10 ml aufweisen, wird es als wünschenswert angesehen, wenn der Strömungswiderstand des Belüftungskanals so geartet ist, dass bei einem Überdruck von 700 mbar im Freiraum zwischen Außenbehälter 2 und Innenbehälter 3 nicht mehr als 0,2 ml/s durch den Belüftungskanal 4 herausströmen. Weiterhin hängt der wünschenswerte Strömungswiderstand auch davon ab, mit welcher Kraft der Außenbehälter 2 bestimmungsgemäß zusammengedrückt wird und welches Medium ausgetragen wird. Weitere Einflussgröße sind die bestimmungsgemäße Betätigungsdauer und die voraussichtliche Zeitspanne zwischen zwei Austragvorgängen.

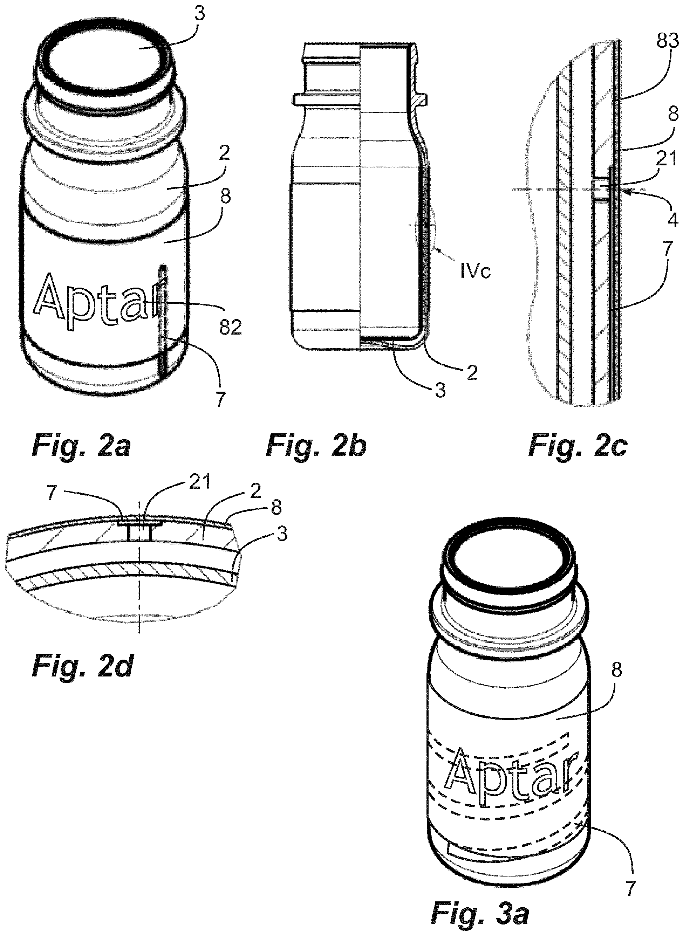

Die Darstellungen der Figuren 2A und 3A zeigen verschiedene Ausgestaltungen des Behälters 1, die für die Erzielung eines solchen Strömungswiderstandes ausgebildet sind.

Die Ausgestaltung der Figuren 2A bis 2D sieht vor, dass wiederum der Außenbehälter 2 von einer vergleichsweise großen Durchtrittsöffnung 21 durchbrochen wird. Diese mündet in eine Vertiefung 7, welche in Axialrichtung sich an der Außenseite des Außenbehälters 2 erstreckt. Damit die Luft beim Ausströmen im Zuge der Betätigung des Spenders nur gegen einen hohen Strömungswiderstand ausströmen kann, ist die nutartige Vertiefung 7 zum überwiegenden Teil durch ein Klebeetikett 8 oder Schrumpfetikett 8 verschlossen. Der Belüftungskanal ist bei dieser Ausgestaltung somit in jenem an der Außenseite des Außenbehälters vorgesehenen Kanalabschnitt umfänglich begrenzt einerseits durch den Außenbehälter 2 und andererseits durch die Innenseite des Etiketts 8. Somit wird eine sehr einfache Möglichkeit geschaffen, einen langen Belüftungskanal hohen Strömungswiderstandes zu schaffen. Hinzu kommt, dass durch eine Variation der Größe des Etikettes auch eine fallweise Einstellung des gewünschten Strömungswiderstandes auf einfache Art und Weise zu realisieren ist.

Der Fig. 3A lässt sich entnehmen, dass durch eine nicht-lineare Erstreckung der nutartigen Vertiefung 7 eine noch deutlich größere Länge des außen liegenden Abschnitts des Belüftungskanals 4 erzielt werden kann. Bei der Variante gemäß Fig. 3A handelt es sich um eine helixförmige nutartige Vertiefung 7, die partiell durch das Etikett 8 umlaufend geschlossen wird.

Es ist vorzugsweise vorgesehen, dass das Etikett 8 nicht nur der Erhöhung des Strömungswiderstandes dient, sondern gleichzeitig jenes Etikett ist, auf dem Informationen 82 zur Erfassung durch den Benutzer aufgedruckt sind.

## Patentansprüche

1. Spender zum Austrag eines pharmazeutischen oder kosmetischen Mediums,
mit
- einem Austragkopf (110) mit Austragöffnung (124) und
- einem Behälter (1) zur Bevorratung des fließfähigen Mediums vor dem Austrag, umfassend
∘ einen elastisch verformbaren Außenbehälter (2) sowie
∘ einen das Medium aufnehmende, formflexiblen Innenbehälter (3), der in einem Innenraum des Außenbehälters (2) angeordnet ist
wobei
- ein Belüftungskanal (4) vorgesehen ist, durch den der Innenraum des Außenbehälters (2) mit einer Umgebung verbunden ist zum Zwecke des Druckausgleichs mittels Ausgleichsluft,
- der Belüftungskanal (4) zumindest abschnittsweise durch ein Etikett (8), insbesondere ein zumindest abschnittsweise selbstklebendes Etikett (8), begrenzt wird,
**dadurch gekennzeichnet, dass**
- der Belüftungskanal (4) zumindest abschnittsweise als Kapillarkanal ausgebildet ist, wobei vorzugsweise der Kapillarkanal (4) eine minimale Querschnittsfläche aufweist, die kleiner als 1 mm² ist, und wobei der Quotient aus der Länge des Kapillarkanals und der mittleren Querschnittsfläche des Kapillarkanals größer als 200mm⁻¹ ist.

2. Spender nach Anspruch 1,
**dadurch gekennzeichnet, dass**
ein Kanalabschnitt des Belüftungskanals (4) gemeinsam gebildet wird durch
- eine nutartig langgestreckte Vertiefung (7) in einem Kanalbauteil (2), wobei das Kanalbauteil (2) vorzugsweise durch den Außenbehälter (2) gebildet wird, und
- das Etikett (8), welches auf dem Kanalbauteil (2) aufgebracht ist,
so dass der Kanalabschnitt durch die Vertiefung (7) und das Etikett (8) umfänglich geschlossen ist.

3. Spender nach einem der vorstehenden Ansprüche,
**dadurch gekennzeichnet, dass**
das Etikett (5, 6, 8) bedruckt ist.

4. Spender nach einem der vorstehenden Ansprüche,
**dadurch gekennzeichnet, dass**
der Belüftungskanal (4) einen Strömungswiderstand aufweist, der so bemessen ist, dass bei einem Zusammendrücken des Außenbehälters (2) ein zum Austrag des Mediums ausreichender Druck im Außenbehälter (2) aufgebaut werden kann, wobei der Belüftungskanals (4) hierfür vorzugsweise einen Strömungswiderstand aufweist, der ausreichend hoch ist, als dass bei einer Druckdifferenz von 700 mbar zwischen dem Außenbehälter und der Umgebung der Volumenstrom 0,2ml/sek nicht übersteigt.

5. Spender nach Anspruch 4,
**dadurch gekennzeichnet, dass**
der Belüftungskanal (4) einen Strömungswiderstand aufweist, der ausreichend gering ist, als dass bei einer Druckdifferenz von 400 mbar zwischen dem Außenbehälter (2) und der Umgebung der Volumenstrom nicht weniger als 0,2ml/sek beträgt.

6. Spender nach einem der vorstehenden Ansprüche,
**dadurch gekennzeichnet, dass**
der Belüftungskanal (4) zumindest abschnittsweise spiralförmig oder helixförmig geformt ist.

## Claims

1. Dispenser for discharging a pharmaceutical or cosmetic medium, with
- a discharge head (110) having a discharge opening (124) and
- a container (1) for storing the flowable medium prior to discharge, comprising
• an elastically deformable outer container (2) and
• a dimensionally flexible inner container (3), which receives the medium and which is arranged in an interior of the outer container (2),
wherein
- a ventilation channel (4) is provided, through which the interior of the outer container (2) is connected to an environment for the purpose of pressure compensation by means of compensating air,
- the ventilation channel (4) is delimited in at least some regions by a label (8), in particular a label (8) that is self-adhesive in at least some regions,
**characterized in that**
- the ventilation channel (4) is designed in at least some regions as a capillary channel, wherein the capillary channel (4) preferably has a minimal cross-sectional surface area that is smaller than 1 mm², and wherein the quotient of the length of the capillary channel and of the mean cross-sectional surface area of the capillary channel is greater than 200 mm⁻¹.

2. Dispenser according to Claim 1, **characterized in that** a channel portion of the ventilation channel (4) is formed jointly by
- a groove-like elongate depression (7) in a channel component (2), wherein the channel component (2) is preferably formed by the outer container (2), and
- the label (8), which is applied to the channel component (2),
such that the channel portion is closed circumferentially by the depression (7) and the label (8).

3. Dispenser according to either of the preceding claims, **characterized in that** the label (5, 6, 8) is printed.

4. Dispenser according to one of the preceding claims, **characterized in that** the ventilation channel (4) has a flow resistance which is dimensioned such that, when the outer container (2) is pressed together, a pressure sufficient to discharge the medium can be built up in the outer container (2), wherein the ventilation channel (4) for this purpose preferably has a flow resistance which is sufficiently high that the volumetric flow rate does not exceed 0.2 ml/sec at a pressure difference of 700 mbar between the outer container and the environment.

5. Dispenser according to Claim 4, **characterized in that** the ventilation channel (4) has a flow resistance which is sufficiently low that the volumetric flow rate is not less than 0.2 ml/sec at a pressure difference of 400 mbar between the outer container (2) and the environment.

6. Dispenser according to one of the preceding claims, **characterized in that** the ventilation channel (4) has a spiral shape or helical shape in at least some regions.

## Revendications

1. Distributeur pour la délivrance d'un milieu pharmaceutique ou cosmétique, avec
- une tête de délivrance (110) avec une ouverture de délivrance (124) et
- un récipient (1) pour stocker le milieu fluide avant la délivrance, comprenant
∘ un récipient extérieur élastiquement déformable (2) ainsi que
∘ un récipient intérieur de forme flexible (3), contenant le milieu, qui est disposé dans un espace intérieur du récipient extérieur (2),
dans lequel
- il est prévu un canal d'aération (4), par lequel l'espace intérieur du récipient extérieur (2) est en communication avec une ambiance en vue d'équilibrer la pression au moyen d'air d'équilibrage,
- le canal d'aération (4) est limité au moins localement par une étiquette (8), en particulier une étiquette au moins localement autoadhésive (8),
**caractérisé en ce que** le canal d'aération (4) est réalisé au moins localement sous forme de canal capillaire, dans lequel le canal capillaire (4) présente de préférence une aire de section transversale minimale, qui est inférieure à 1 mm², et dans lequel le quotient de la longueur du canal capillaire par l'aire moyenne de section transversale du canal capillaire est supérieur à 200 mm⁻¹.

2. Distributeur selon la revendication 1, **caractérisé en ce qu'**une partie de canal du canal d'aération (4) est formée en commun par
- un creux allongé en forme de rainure (7) dans un composant de canal (2), dans lequel le composant de canal (2) est formé de préférence par le récipient extérieur (2), et
- l'étiquette (8), qui est apposée sur le composant de canal (2),
de telle manière que la partie de canal soit fermée en périphérie par le creux (7) et l'étiquette (8).

3. Distributeur selon l'une quelconque des revendications précédentes, **caractérisé en ce que** l'étiquette (5, 6, 8) est imprimée.

4. Distributeur selon l'une quelconque des revendications précédentes, **caractérisé en ce que** le canal d'aération (4) présente une résistance à l'écoulement, qui est calculée de telle manière que lors d'une compression du récipient extérieur (2) une pression suffisante pour la délivrance du milieu puisse être créée dans le récipient extérieur (2), dans lequel le canal d'aération (4) présente à cet effet de préférence une résistance à l'écoulement, qui est suffisamment élevée pour que, pour une différence de pression de 700 mbar entre le récipient extérieur et l'ambiance, le débit volumique ne dépasse pas 0,2 ml/s.

5. Distributeur selon la revendication 4, **caractérisé en ce que** le canal d'aération (4) présente une résistance à l'écoulement, qui est suffisamment faible pour que, pour une différence de pression de 400 mbar entre le récipient extérieur (2) et l'ambiance, le débit volumique ne soit pas inférieur à 0,2 ml/s.

6. Distributeur selon l'une quelconque des revendications précédentes, **caractérisé en ce que** le canal d'aération (4) est réalisé au moins localement en forme de spirale ou en forme d'hélice.
